(19) Europäisches Patentamt
European Patent Office
Office européen des brevets

(11) **EP 3 344 115 B1**

(12) **FASCICULE DE BREVET EUROPEEN**

(45) Date de publication et mention de la délivrance du brevet:
**17.11.2021 Bulletin 2021/46**

(21) Numéro de dépôt: **16770068.1**

(22) Date de dépôt: **31.08.2016**

(51) Int Cl.:
*A61B 3/103* $^{(2006.01)}$  *A61B 3/04* $^{(2006.01)}$

(86) Numéro de dépôt international:
**PCT/FR2016/052160**

(87) Numéro de publication internationale:
**WO 2017/037386 (09.03.2017 Gazette 2017/10)**

(54) **EQUIPEMENT D'OPTOMETRIE, ENSEMBLE ET SYSTEME COMPRENANT UN TEL EQUIPEMENT**

OPTOMETRIEVORRICHTUNG, ANORDNUNG UND SYSTEM MIT SOLCH EINER VORRICHTUNG

OPTOMETRY APPARATUS, ASSEMBLY AND SYSTEM COMPRISING SUCH AN APPARATUS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.09.2015 FR 1558191**

(43) Date de publication de la demande:
**11.07.2018 Bulletin 2018/28**

(73) Titulaire: **ESSILOR INTERNATIONAL**
**92440 Charenton-Le-Pont (FR)**

(72) Inventeurs:
- **NAUCHE, Michel**
  **94227 Charenton-le-Pont Cedex (FR)**
- **BOUTINON, Stéphane**
  **94227 Charenton-le-Pont Cedex (FR)**

(74) Mandataire: **Jacobacci Coralis Harle**
**32, rue de l'Arcade**
**75008 Paris (FR)**

(56) Documents cités:
EP-A2- 2 645 165    WO-A1-92/19149
WO-A1-2015/107303   JP-A- 2001 340 296
US-A1- 2004 032 568

**Description**

DOMAINE TECHNIQUE AUQUEL SE RAPPORTE L'INVENTION

La présente invention concerne le domaine de l'optométrie.

Elle concerne plus particulièrement un équipement d'optométrie, ainsi qu'un ensemble et un système comprenant un tel équipement.

ARRIERE-PLAN TECHNOLOGIQUE

Dans le cadre de la mesure de l'acuité visuelle d'un patient, il a déjà été proposé de simuler la compensation visuelle à fournir, par exemple au moyen de lunettes d'essai ou d'un réfracteur, tel qu'une tête de réfraction.

Dans la tête de réfraction, des verres d'essai sont placés sur plusieurs disques, entraînés en rotation manuellement ou à l'aide d'un mécanisme motorisé. On comprend toutefois qu'un tel objet présente un encombrement et un poids importants, liés au nombre de verres placés sur chaque disque.

Les lunettes d'essais sont moins encombrantes. On prévoit en effet qu'elles accueillent successivement des verres d'essai ayant des corrections différentes, jusqu'à trouver la correction qui convient au patient.

Cette solution est toutefois assez peu pratique, notamment parce qu'elle nécessite le stockage séparé des verres d'essai dans des boîtes dédiées. Elle implique en outre des changements de lentilles qui provoquent des transitions de puissance de correction non désirées et non continues.

Le document WO 2015/107 303 décrit un système de compensation visuelle comprenant deux lentilles cylindriques montées en rotation autour d'un axe optique et une lentille de puissance sphérique variable.

Le document EP 1 138 253 décrit des lunettes d'essai comprenant une lentille de type Alvarez et une paire de lentilles cylindriques.

OBJET DE L'INVENTION

Dans ce contexte, la présente invention propose un équipement d'optométrie conforme à la revendication 1.

L'équipement d'optométrie comprend ainsi le moteur qui permet de régler, par action sur l'organe de réglage, la correction obtenue grâce aux lunettes. Les lunettes s'en trouvent allégées d'autant et sont ainsi bien adaptées à être portées pour un essai de la correction choisie.

Selon d'autres caractéristiques optionnelles et donc non limitatives :

- un module de communication est conçu pour recevoir une consigne en provenance d'un appareil électronique extérieur ;
- un module de commande est conçu pour commander le moteur d'entraînement en fonction de la consigne reçue ;
- l'équipement d'optométrie comprend un afficheur et un imageur disposés de part et d'autre d'une région de réception des lunettes de compensation visuelle ;
- l'équipement d'optométrie comprend un module d'analyse d'une image de l'afficheur générée par l'imageur et un module de commande du moteur d'entraînement en fonction de données générées par le module d'analyse.

L'invention propose également un ensemble conforme à la revendication.

Selon d'autres caractéristiques optionnelles et donc non limitatives :

- les lunettes de compensation visuelle comprennent au moins une lentille ayant un axe optique ;
- les lunettes de compensation visuelle sont conçues pour modifier, en cas de mouvement de l'organe de réglage, une puissance sphérique selon l'axe optique générée par ladite lentille ;
- les lunettes de compensation visuelle sont conçues pour modifier, en cas de mouvement de l'organe de réglage, une correction cylindrique selon l'axe optique générée par ladite lentille ;
- les lunettes de compensation visuelle sont conçues pour modifier, en cas de mouvement de l'organe de réglage, une puissance de ladite correction cylindrique ;
- les lunettes de compensation visuelle sont conçues pour modifier, en cas de mouvement de l'organe de réglage, un axe de ladite correction cylindrique.

L'invention propose enfin un système comprenant un équipement d'optométrie tel que mentionné ci-dessus et ledit appareil électronique extérieur, caractérisé en ce que ledit appareil électronique extérieur est un autre appareil d'optométrie, par exemple un auto-réfractomètre ou un réfracteur.

On peut prévoir que ledit appareil électronique extérieur détermine ladite consigne en fonction d'une mesure d'amétropie ou d'astigmatisme.

## DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 représente un ensemble optique comprenant une lentille de puissance cylindrique variable et une lentille de puissance sphérique variable ;
- la figure 2 représente une vue en coupe de l'ensemble optique de la figure 1 ;
- la figure 3 représente un écorché en perspective de l'ensemble optique de la figure 1;
- la figure 4 représente des lunettes de compensation visuelle comprenant deux tels ensembles optiques ;
- la figure 5 représente un équipement de réception des lunettes de la figure 4 ;
- la figure 6 représente les lunettes de compensation visuelle de la figure 4 reçues dans l'équipement de réception de la figure 5 ;
- la figure 7 représente schématiquement les principaux composants électroniques de l'équipement de réception de la figure 5 ; et
- la figure 8 représente une variante envisageable pour la réalisation d'au moins un ensemble optique des lunettes de compensation visuelle de la figure 4.

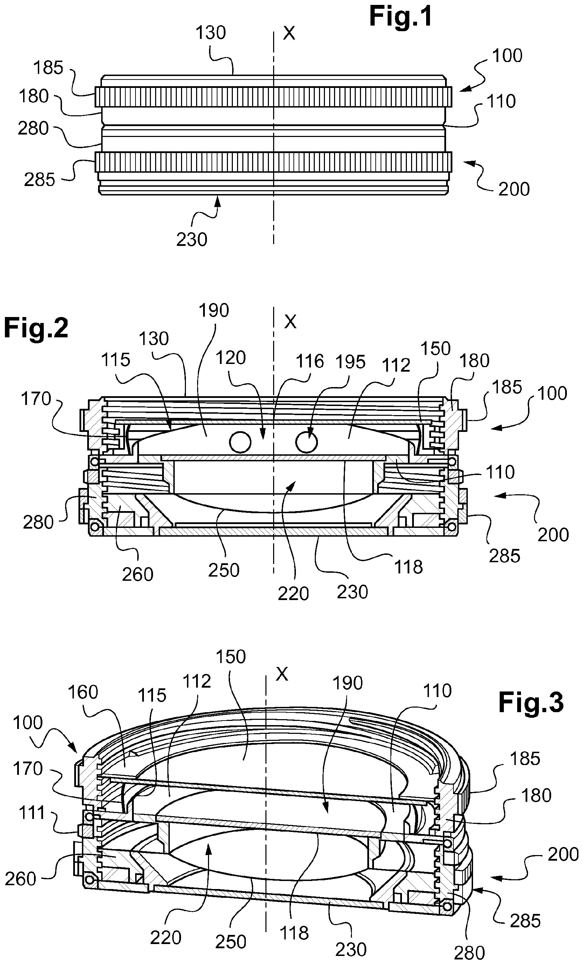

Les figures 1 à 3 représentent un ensemble optique comprenant une première lentille 100 et une seconde lentille 200.

La première lentille 100 et la seconde lentille 200 sont ici réalisées respectivement de part et d'autre d'un même bâti (ou corps) 110. Une plaque de séparation indéformable (transparente) 118, montée sur le bâti commun 110, délimite des chambres remplies de liquide de la première lentille 100 et de la seconde lentille 200, comme expliqué plus loin, et permet ainsi un fonctionnement indépendant des deux lentilles 100, 200.

En variante, l'une ou l'autre des deux lentilles 100, 200 pourrait être réalisée séparément. On décrit d'ailleurs ci-dessous chaque lentille 100, 200 séparément.

Comme expliqué dans la suite, la première lentille 100 est conçue pour générer, selon un axe optique X de la première lentille, une puissance optique cylindrique variable.

La seconde lentille 200 est quant à elle conçue pour générer, selon son axe optique identique à l'axe optique X de la première lentille 100, une puissance optique sphérique variable.

La première lentille 100 comprend le bâti (ou corps) 110 et une lame transparente flexible 150.

Le bâti 110 présente une ouverture centrale 120 fermée à une extrémité par une première lame de fermeture (transparente) 130 et à l'autre extrémité par la plaque de séparation 118 ; la lame transparente flexible 150 est située dans l'ouverture centrale 120, entre la première lame de fermeture 130 et la plaque de séparation 118.

Deux parois intégrées au bâti 110 permettent un guidage de la lame flexible 150 destiné à empêcher toute rotation de la lame flexible 150 autour de l'axe optique X.

Le bâti 110 présente une arête 115 qui s'étend à la périphérie de l'ouverture centrale 120 et qui est inscrite dans une surface cylindrique dont l'axe est perpendiculaire et sécant à l'axe optique X.

Le bâti 110 comprend par exemple à cet effet une paroi 112 qui entoure l'ouverture centrale 120 et dont le bord d'extrémité libre définit l'arête 115 précitée.

L'arête 115 a pour fonction de créer une butée servant de point de départ à la cambrure de la lame flexible 150 (par flexion), comme expliqué plus loin. La forme cylindrique de l'arête 150 (c'est-à-dire la surface cylindrique dans laquelle l'arête 115 est inscrite) a de ce fait un rayon inférieur au rayon correspondant à la puissance cylindrique maximum souhaitée, typiquement de l'ordre de 40 mm ou moins.

La première lentille 100 comprend également une première pièce de commande 160 solidaire de la lame flexible 150

La première pièce de commande 160 est ici réalisée sous la forme d'une plaque de commande annulaire entourant l'ouverture centrale 120 et comportant, au niveau de chacune de deux zones opposées à 180°, un filet externe.

La première pièce de commande 160 est montée dans le bâti 110 guidée en translation selon l'axe optique X.

La première lentille 100 comprend également une première membrane élastique 170 de forme cylindrique et reliant le bâti 110 et la première pièce de commande 160.

La lame flexible 150, la première pièce de commande 160, la première membrane élastique 170, la plaque intermédiaire 118 et le bâti 110 forment une chambre remplie d'un liquide 190. Ici, ce liquide 190 est identique à celui utilisé dans le document US8000022, à savoir de l'huile siliconée (par exemple du type Rhodrosil Huile 47).

Le bâti 110 comporte des ouvertures de passage de fluide 195 dans la paroi 112 susmentionnée afin de faciliter le passage du liquide entre les différentes parties de la chambre.

La première membrane élastique 170 est conçue pour se déformer (par étirement notamment) afin de compenser les variations de volume de la chambre remplie du liquide 190 causées par la flexion de la lame flexible 150, comme décrit ci-après. Comme bien visible sur les figures 2 et 3, la première membrane élastique 170 est située à la périphérie du bâti 110 et ne joue donc aucun rôle optique.

La première lentille cylindrique 100 comprend enfin une première bague de commande 180, mobile uniquement en rotation sur un angle donné autour de l'axe optique X. La première bague de commande 180 comporte un filetage interne (ici de section carré) centré sur l'axe optique X et qui engrène avec le filet externe de la première pièce de commande 160.

La première bague de commande 180 présente également une pluralité de dents 185 sur toute sa périphérie de façon à former une roue dentée et à pouvoir ainsi être entraînée en rotation par un premier système d'entraînement (non représenté), par exemple un moteur dont l'axe de sortie présente une vis sans fin qui coopère avec les dents 185 de la première bague de commande 180.

On peut prévoir par ailleurs de limiter la course de la première bague de commande 180, par exemple au moyen d'une butée franche réalisée entre le bâti 110 et la première bague de commande 180.

On décrit à présent les mouvements qui permettent la flexion de la lame flexible 150 et ainsi l'obtention d'une puissance optique cylindrique variable.

On considère pour l'exposé que la première pièce de commande 160 est dans sa position la plus haute (en considérant l'orientation de la figure 2), c'est-à-dire la plus proche de la première lame de fermeture 130, comme représenté sur les figures 2 et 3. Comme visible sur ces figures, dans cette position de la première pièce de commande 160, la lame flexible 150 n'est pas en contact avec l'arête 115.

Lors de la mise en rotation de la première bague de commande 180 (par exemple au moyen du premier système d'entraînement précité), la première pièce de commande 160 entre en translation (vers le bas sur la figure 2) par l'intermédiaire du système vis/écrou, sur quelques degrés, jusqu'à ce que la lame flexible 150 vienne en contact avec l'arête 115 de forme cylindrique solidaire du bâti 110 (ce contact s'effectuant en premier lieu au sommet 116 de l'arête 115 sur la figure 2, c'est-à-dire dans le plan contenant l'axe optique X et l'axe de la surface cylindrique contenant l'arête 115).

En continuant la rotation de la première bague de commande 180, l'ensemble formé de la première pièce de commande 160 et de la lame flexible 150 poursuit sa translation (vers le bas en figure 2, c'est-à-dire en s'éloignant de la première lame de fermeture 130) de sorte que la lame flexible 150 entre en flexion progressive, avec une valeur de rayon (de flexion) directement liée à l'angle de rotation de la première bague de commande 180.

Grâce à l'arête de forme cylindrique, la lame flexible 150 va se déformer préférentiellement selon une forme cylindrique, ce qui permet d'obtenir la correction cylindrique recherchée, laquelle a une puissance variable selon la valeur du rayon de flexion précité.

Par ailleurs, du fait que la déformation de la lame flexible est initiée par l'arête 150 inscrite dans une surface cylindrique, l'axe de cette correction cylindrique correspond à l'axe de cette surface cylindrique (soit un axe fixe par rapport au bâti 110 et perpendiculaire à l'axe optique X).

On remarque que la lame flexible 150 subit en général également une seconde déformation plus ou moins importante en fonction notamment de son anisotropie, de sa raideur en flexion et de l'augmentation de pression liée à la déformation de la première membrane élastique 170. De ce fait, la lame flexible 150 n'a en général pas une forme purement cylindrique, mais une forme torique.

La composante sphérique additionnelle pourra alors éventuellement être compensée par d'autres moyens optiques, ici par action sur la seconde lentille 200 décrite plus bas.

Lors de la déformation de la lame flexible 150, le volume au sein de la chambre remplie du liquide 190 demeure constant. En effet, l'augmentation de pression générée par la déformation de la lame flexible 150 entraîne la déformation de la première membrane élastique 170 de manière à absorber le transfert de liquide 190 issu de la zone utile.

On remarque par ailleurs que cette légère mise en pression (réalisée par la lame flexible 150) permet d'assurer une réversibilité du mouvement, tout en assurant un calage du jeu au sein du système vis/écrou.

En résumé, par entraînement de la première bague de commande 180 en rotation au moyen du premier système d'entraînement, on fait varier comme expliqué ci-dessus la puissance cylindrique de la première lentille 100 (l'axe de la correction cylindrique étant en revanche fixe par rapport au bâti 110 comme expliqué plus haut).

La seconde lentille 200 comprend un support présentant une ouverture centrale 220, une seconde membrane élastique 250 reçue dans l'ouverture centrale 220, une seconde pièce de commande 260 solidaire de la seconde membrane élastique 250 et une seconde bague de commande 280.

Le support de la seconde lentille 200 est ici solidaire du bâti 110 de la première lentille 110, par exemple réalisé d'une pièce avec celui-ci.

La seconde pièce de commande 260 est montée guidée en translation dans le support, ici au moyen de trois colonnes de guidage.

La seconde bague de commande 280 est mobile uniquement en rotation autour de l'axe optique X et permet, lors de ses mouvements de rotation, de déplacer la seconde pièce de commande 260 en translation selon l'axe optique X au moyen d'un système vis/écrou (formé par exemple d'un filetage interne de la seconde bague de commande 280 qui coopère avec un filetage externe de la seconde pièce de commande 260).

La seconde membrane élastique 250, la seconde pièce de commande 260, la plaque de séparation 118 et le support (ici le bâti commun 110) forment une chambre remplie de liquide de sorte que le déplacement en translation de la seconde pièce de commande 260 entraîne une déformation élastique (avec étirement) de la seconde membrane élastique 250, laquelle adopte ainsi une forme essentiellement sphérique, avec un rayon de courbure dépendant de la position de la seconde pièce de commande 260.

On obtient ainsi la puissance sphérique variable recherchée.

La seconde bague de commande 280 porte sur toute sa périphérie une pluralité de dents 285 de façon à former une roue dentée et à pouvoir ainsi être entraînée en rotation par un second système d'entraînement (non représenté), par exemple un moteur dont l'axe de sortie présente une vis sans fin qui coopère avec les dents 285 de la seconde bague de commande 280.

Ainsi, par entraînement de la seconde bague de commande 280 en rotation au moyen du second système d'entraînement, on fait varier comme expliqué ci-dessus la puissance sphérique de la seconde lentille 200.

L'ensemble de la cinématique est sans jeu de construction. Grâce à cette caractéristique, il est possible d'établir une loi de commande de la cinématique qui est continue. En effet, dans le cas contraire, puisque le sens des efforts s'exerçant sur la cinématique s'inverse lorsque la membrane passe d'une forme convexe à une forme concave, un jeu de construction risquerait de fausser la loi de commande ou compliquerait l'élaboration de cette loi de commande.

Comme décrit ci-dessus, il est possible de faire varier la puissance cylindrique et la puissance sphérique de la correction obtenue par l'ensemble optique formé de la première lentille 100 et de la seconde lentille 200 en entraînant respectivement en rotation la première bague de commande 180 et la seconde bague de commande 280.

Afin de faire varier l'axe de cylindre de la correction cylindrique obtenue par la première lentille 100, l'ensemble optique précité peut être monté mobile en rotation autour de l'axe optique X par rapport à un référentiel fixe (notamment par rapport à l'œil du patient devant lequel l'ensemble optique est placé dans le cadre d'un examen de réfraction subjective).

On prévoit alors par exemple pour ce faire un ensemble de dents 111 prévues sur la paroi externe du bâti 110, sur la périphérie de l'ensemble optique.

On peut prévoir que la rotation du bâti 110 par rapport au référentiel fixe entraîne un mouvement de rotation du bâti d'une part par rapport à la première bague de commande 180 et d'autre part par rapport à la seconde bague de commande 280 (ces deux bagues de commande 180, 280 étant immobiles dans le référentiel fixe en l'absence d'entraînement par les premier et second systèmes d'entraînement).

On prévoit dans ce cas (lorsque l'on souhaite faire varier seulement l'axe de cylindre de la correction cylindrique) d'entraîner en rotation les bagues de commande 180, 280 (simultanément ou non à la rotation du bâti 110) de manière à conserver la position relative du bâti 110, de la première bague de commande 180 et de la seconde bague de commande 280 afin de conserver les valeurs de puissance cylindrique et de puissance sphérique souhaitées.

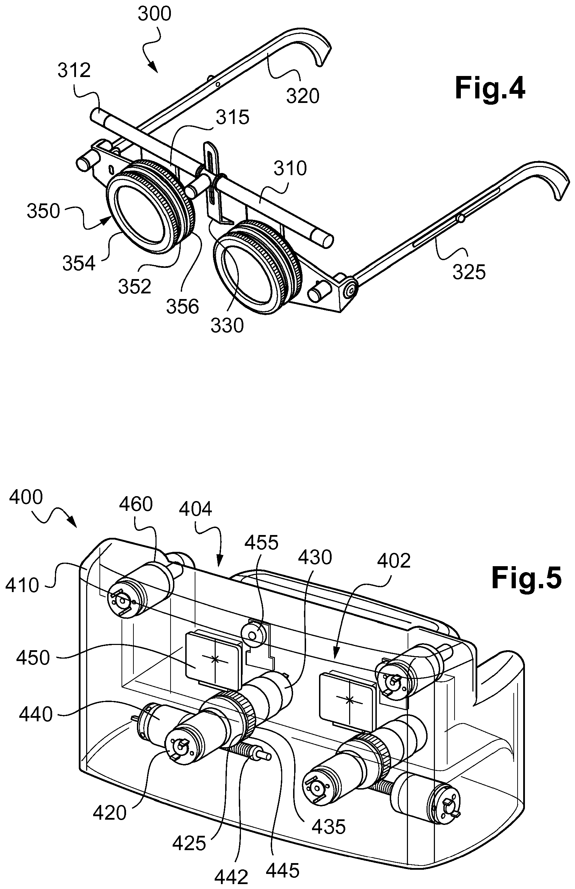

La figure 4 représente des lunettes de compensation visuelle 300 comprenant deux ensembles optiques 350 tels que décrits ci-dessus en référence aux figures 1 à 3 montés sur une monture.

La monture comprend une traverse 310 qui porte deux éléments de support 315 destinés chacun à accueillir un ensemble optique 350.

La position de chaque élément de support 315 sur la traverse 310 est réglable en translation selon l'axe de la traverse 310, ici par action sur un pignon 312 disposé à une extrémité correspondante de la traverse 310, de manière à pouvoir ajuster la position de l'ensemble optique 350 concerné à la demi-distance interpupillaire du porteur des lunettes de compensation visuelle 300.

Chaque élément de support 315 porte un ensemble optique 350, en laissant à cet ensemble optique 350 une possibilité de rotation autour de son axe lorsqu'il est entraîné en rotation par un système d'entraînement extérieur, comme décrit ci-après, au moyen de la roue dentée 352 formée autour de son bâti comme indiqué plus haut (voir notamment les dents 111 en figure 3).

Chaque ensemble optique 350 est par ailleurs monté sur l'élément de support 315 correspondant de manière à laisser accessible (dans une région au moins) la première bague de commande 354 (correspondant à la première bague de commande 180 sur les figures 1 à 3) et la seconde bague de commande 356 (correspondant à la seconde bague de commande 280 sur les figures 1 à 3).

On peut d'ailleurs éventuellement prévoir, pour chaque ensemble 350, un capotage (non représenté) qui recouvre les bagues de commande 354, 356 (de manière à rendre impossible une manipulation de ces dernières par un utilisateur) sauf aux endroits où les bagues de commande 354, 356 coopèrent avec les éléments de transmission 425, 435 comme expliqué plus loin.

Chaque élément de support 315 porte par ailleurs une branche 320 de la monture des lunettes de compensation visuelle 300, avec une possibilité d'orientation de la branche 320 concernée par rapport à l'élément de support 315 afin de pouvoir ajuster l'orientation de la branche 320 à l'angle pantoscopique du porteur.

Chaque branche 320 comporte par ailleurs un mécanisme 325 de réglage de longueur de la branche concernée.

La traverse 310 porte par ailleurs un support nasal 330. La position (en hauteur) du support nasal 330 est réglable afin de s'ajuster à la morphologie du porteur.

La figure 5 représente un équipement de réception 400 des lunettes qui viennent d'être décrites.

Un tel équipement de réception 400 comprend un socle 410, ici de forme générale parallélépipédique, présentant un logement 402, ouvert ici vers le haut et dimensionné de manière à accueillir les lunettes de compensation visuelle 300, en particulier la traverse 310, les éléments de support 315 et les ensembles optiques 350.

Le socle 410 présente également des ouvertures 404, formées dans une paroi latérale du logement 402, pour le passage des branches 320 des lunettes de compensation visuelle 300.

Pour chaque ensemble optique 350, l'équipement de réception 400 comprend :

- un premier moteur (électrique) d'entraînement 420 d'un premier élément de transmission 425 (ici une roue dentée placée sur l'arbre de sortie du premier moteur 420) ;
- un second moteur (électrique) d'entraînement 430 d'un second élément de transmission 435 (ici une roue dentée placée sur l'arbre de sortie du second moteur 430) ;
- un troisième moteur (électrique) d'entraînement 440 d'un troisième élément de transmission 445 (ici une roue dentée entraînée par une vis 442 montée sur l'arbre de sortie du troisième moteur 440).

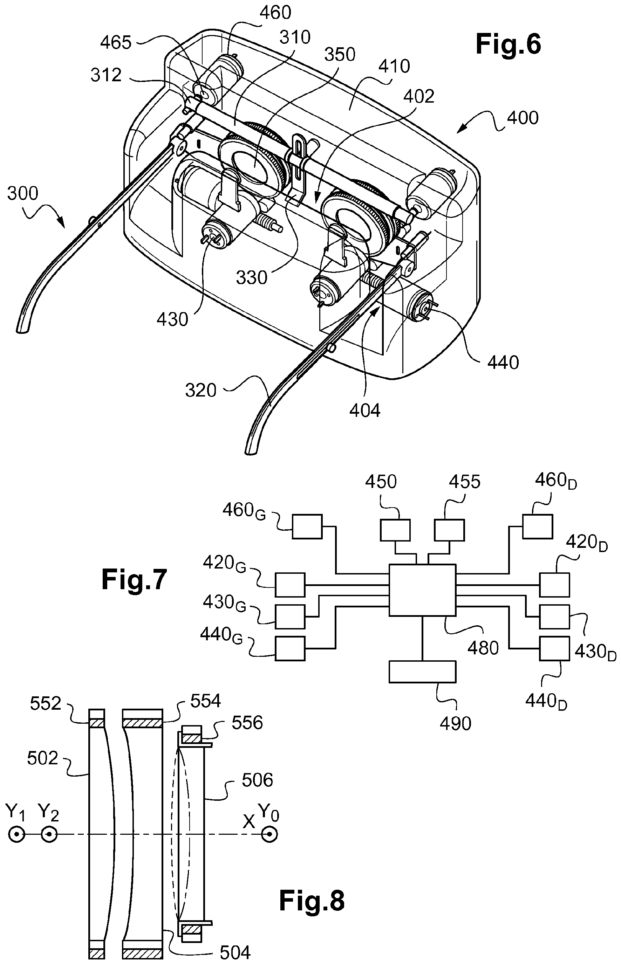

Chaque élément de transmission 425, 435, 445 est placé dans l'équipement de réception (ici dans une ouverture d'une paroi de fond du logement 402 formé dans le socle 410) de manière à coopérer avec un organe de commande 354, 356, 352 de l'ensemble optique 350 concerné lorsque les lunettes de compensation visuelle 300 sont reçues dans l'équipement de réception 400, comme représenté en figure 6.

Précisément, lorsque les lunettes de compensation visuelle 300 sont reçues dans le socle 410 (positionnées dans le logement 402, avec les branches 320 traversant les ouvertures 404) :

- le premier élément de transmission 425 coopère avec la première bague de commande 354 (de sorte que le premier moteur d'entraînement 420 et le premier élément de transmission 425 forment le premier système d'entraînement mentionné plus haut) ;
- le second élément de transmission 435 coopère avec la seconde bague de commande 356 (de sorte que le second moteur d'entraînement 430 et le second élément de transmission 435 forment le second système d'entraînement mentionné plus haut) ;
- le troisième élément de transmission 445 coopère avec la roue dentée 352 (de sorte que le troisième moteur d'entraînement 440 et le troisième élément de transmission 445 forment le troisième système d'entraînement mentionné plus haut).

Ainsi, par action respectivement du premier moteur d'entraînement 420, du second moteur d'entraînement 430 et du troisième moteur d'entraînement 440, on peut régler la puissance cylindrique, la puissance sphérique et l'axe de la correction cylindrique générés par l'ensemble optique 350 concerné.

Dans l'exemple décrit ici, l'équipement de réception 400 comprend également pour chaque ensemble optique 350 à recevoir dans le logement 402, un système de contrôle de la puissance optique comprenant un afficheur 450 et un imageur (par exemple une caméra 455) situés respectivement de part et d'autre du logement 402 dans une région destinée à recevoir un ensemble optique 350.

Comme également expliqué plus bas, un circuit de commande 480 (ici inclus dans l'équipement de réception 400) est conçu pour déterminer les caractéristiques de la correction optique fournie par l'ensemble optique 350 en fonction d'au moins une image de l'afficheur 450 observé à travers l'ensemble optique 350 par la caméra 455, par calcul de déflectométrie.

L'équipement de réception 400 comprend enfin, en association avec chacun des deux côtés de la monture des lunettes de compensation visuelle 300, un quatrième moteur (électrique) d'entraînement 460 d'un quatrième élément de transmission 465.

Ce quatrième élément de transmission 465 est positionné dans l'équipement de réception 400 (c'est-à-dire dans le socle 410) de manière à coopérer avec un pignon 312 correspondant de manière à régler (par action du quatrième moteur d'entraînement 460) la position de l'élément de support 315 correspondant relativement à la traverse 310.

La figure 7 représente schématiquement les principaux composants électroniques de l'équipement de réception 400.

Le circuit de commande 480 susmentionné comprend par exemple un processeur (par exemple un microprocesseur) et une ou plusieurs mémoire(s) qui mémorisent notamment des instructions de programme d'ordinateur entraînant la mise en œuvre, par le circuit de commande 480, des procédés décrits ci-dessous lorsque ces instructions sont exécutées par le processeur.

Des données d'étalonnage des deux modules 350 peuvent en outre être stockées dans une des mémoires susmentionnées du circuit de commande 480.

En variante, le circuit de commande 480 pourrait être réalisé sous la forme d'un circuit intégré à application spécifique.

L'équipement de réception 400 comprend également un module de communication 490, relié au circuit de commande 480 et conçu pour établir un canal de communication avec un autre appareil électronique (non représenté), par exemple un auto-réfractomètre ou un réfracteur.

Le module de communication 490 est par exemple conçu pour établir une liaison sans fil avec l'autre appareil électronique, ici en se connectant à un réseau local sans fil (ou WLAN pour "*Wireless Local Area Network*") auquel est également connecté l'autre appareil électronique. En variante, le module de communication 490 peut établir une communication avec l'autre appareil électronique via une liaison filaire.

Le circuit de commande 480 est également relié aux premiers moteurs d'entraînement $420_G$, $420_D$, aux seconds moteurs d'entraînement $430_G$, $430_D$, aux troisièmes moteurs d'entraînement $440_G$, $440_D$ et aux quatrièmes moteurs d'entraînement $460_G$, $460_D$ (les moteurs d'entraînements destinés à commander l'ensemble optique associé à l'œil gauche étant référencés avec la lettre G en indice, tandis que les moteurs d'entraînements destinés à commander l'ensemble optique associé à l'œil droit sont référencés avec la lettre D en indice).

Le circuit de commande 480 est enfin relié à l'afficheur 450 et à la caméra 455, comme déjà indiqué.

On décrit à présent un exemple d'utilisation du système qui vient d'être décrit.

Le circuit de commande 480 reçoit des informations de consigne en provenance de l'autre appareil électronique, via le canal de communication établi par le module de communication 490. Ces informations de consignes comprennent par exemple, pour chaque œil, une consigne de puissance sphérique S, une consigne de puissance cylindrique C, une consigne d'angle de correction cylindrique $\alpha$ et une consigne de demi-distance inter pupillaire PD.

Ces consignes correspondent par exemple à des valeurs (notamment des valeurs d'amétropie et physiologiques) mesurées sur le porteur à l'aide de l'autre appareil électronique (auto-réfractomètre ou réfracteur).

On considère que les lunettes de compensation visuelle 300 sont alors placées dans l'équipement de réception 400, comme représenté en figure 6.

Le circuit de commande 480 pilote alors les quatrièmes moteurs d'entraînement $460_G$, $460_D$ de manière à placer chacun des ensembles optiques 350 au droit du système de contrôle de puissance optique correspondant (afficheur 350, caméra 355).

Pour chaque œil, le circuit de commande 480 pilote ensuite le premier moteur d'entraînement 420, le second moteur d'entraînement 430, le troisième moteur d'entraînement 440 de manière à ce que l'ensemble optique 350 concerné génère une correction optique dont les caractéristiques (puissance sphérique, puissance cylindrique, axe du cylindre) soient conformes aux informations de consigne reçues pour l'œil concerné.

Dans l'exemple décrit ici, le circuit de commande 480 détermine à chaque instant les caractéristiques de la correction effective au moyen de l'afficheur 450 et de la caméra 455 (par analyse des images de l'afficheur 450 formées à travers l'ensemble optique 350 sur la caméra 455) et commande la rotation du moteur d'entraînement 420, 430, 440 concerné jusqu'à obtenir la valeur de consigne.

En variante, le circuit de commande 480 peut être conçu pour déterminer, par un traitement approprié des informations de consigne reçues (utilisant notamment les données d'étalonnage mentionné plus haut), des consignes de position pour chacun des organes de commande 354, 356, 352 de l'ensemble optique 350, et pour piloter le premier moteur d'entraînement 420, le second moteur d'entraînement 430, le troisième moteur d'entraînement 440 de manière à atteindre ces consignes de positions respectives.

On peut prévoir dans ce cas trois référentiels sur chaque ensemble optique 350 (respectivement pour le réglage de puissance sphérique, le réglage de puissance cylindrique et le réglage de l'axe de cylindre), identifiables au moyen de capteurs solidaires de l'équipement de réception 400, de manière à contrôler les caractéristiques de la correction optique directement à partir des moteurs d'entraînement (du fait que l'on connaît la position de chaque organe de commande 352, 354, 356 dans son référentiel précité).

Une fois la correction souhaitée obtenue pour chaque ensemble optique 350, le circuit de commande 480 pilote également (pour chaque côté) le quatrième moteur d'entraînement 460 de manière à ajuster la position de l'ensemble optique 350 concerné à la demi-distance interpupillaire PD souhaitée (i.e. reçue en tant que consigne comme indiqué plus haut).

Les lunettes de compensation visuelle 300 peuvent alors être retirées de l'équipement de réception 400 et être utilisées par le porteur, ici pour vérifier la validité des mesures faites au niveau de l'autre appareil électronique.

La figure 8 représente une variante envisageable pour la réalisation d'au moins un ensemble optique des lunettes de compensation visuelle 300.

Un tel ensemble optique comprend :

- une lentille plan-cylindre convexe 502, de puissance cylindrique $C_0$ selon l'axe optique X, montée sur une première roue dentée 552 déplaçable en rotation autour de l'axe optique X ;
- une lentille plan-cylindre concave 504, de puissance cylindrique négative $-C_0$ selon l'axe optique X, montée sur une seconde roue dentée 554 déplaçable en rotation autour de l'axe optique X ; et
- une lentille 506 de puissance sphérique (selon l'axe optique) $S_v$ variable par rotation d'une troisième roue dentée 506 (la troisième roue dentée 506 coopérant par un système de type vis-écrou avec un élément de commande du rayon d'une membrane de la lentille 506, comme expliqué plus haut pour la lentille 200).

La valeur absolue (ou module), ici $C_0$, de la puissance cylindrique (ici $-C_0$) de la lentille plan-cylindre concave 504 est donc égale à la valeur absolue ($C_0$) (ou module) de la puissance cylindrique ($C_0$) de la lentille plan-cylindre convexe 502.

Chacune des trois lentilles 502, 504, 506 comporte une première face plane, perpendiculaire à l'axe optique X, et une seconde face, opposée à la première face et optiquement active :

- la face optiquement active de la lentille 502 est de forme cylindrique convexe (l'axe $Y_1$ du cylindre définissant cette face étant perpendiculaire à l'axe optique X) ;
- la face optiquement active de la lentille 504 est de forme cylindrique concave (l'axe $Y_2$ du cylindre définissant cette face étant perpendiculaire à l'axe optique X) ;
- la face optiquement active de la lentille 506 de puissance sphérique variable $S_v$ est déformable et peut ainsi prendre une forme sphérique convexe (comme illustré en pointillés sur la figure 8), une forme plane (comme illustré par un trait plein sur la figure 8) ou une forme sphérique concave (comme illustré en trait mixte sur la figure 8).

La lentille 506 de puissance sphérique variable $S_V$ est par exemple une lentille du type décrit dans le document EP 2 034 338. Une telle lentille comprend une cavité fermée par une membrane déformable transparente et une paroi plane transparente mobile ; la cavité contient un liquide transparent de volume constant qui est plus ou moins contraint par la face mobile, afin de déformer la membrane qui est de ce fait soit une surface concave sphérique, soit une surface plane, soit une surface convexe sphérique. Dans la lentille utilisée, une transformation de mouvement réalisée par un système vis écrou permet d'assurer la transformation de mouvement translation - rotation. Ainsi, une rotation de la route dentée 556 entraîne en translation l'élément de commande susmentionné, ce qui provoque la déformation de la membrane transparente comme expliqué par exemple dans le document EP 2 034 338 précité. On peut ainsi faire varier continûment la puissance sphérique $S_v$ par action mécanique sur la lentille 506. Dans l'exemple décrit ici, la lentille 506 a une focale variable entre -40 mm et 40 mm, soit une puissance sphérique $S_v$ variable entre -25D et 25D (D étant la dioptrie, unité de mesure de la vergence, inverse de la focale exprimée en mètres).

Par ailleurs, les lentilles plan-cylindre 502, 504 ont respectivement comme déjà indiqué une puissance cylindrique $-C_0$ et $C_0$, ici avec $C_0 = 5D$.

Par la rotation de la roue dentée 552, l'axe $Y_1$ du cylindre convexe formé sur la face optiquement active de la lentille plan-cylindre convexe 502 peut former un angle variable $\alpha_1$ avec un axe de référence $Y_0$ (fixe et perpendiculaire à l'axe optique X).

De même, par rotation de la roue dentée 554, l'axe $Y_2$ du cylindre concave formé sur la face optiquement active de la lentille plan-cylindre concave 4 peut former un angle variable $\alpha_2$ avec l'axe de référence $Y_0$.

Par calcul de la vergence sur les différents méridiens, on obtient les formules suivantes pour la puissance sphérique S, la puissance cylindrique C et l'angle d'astigmatisme $\alpha$ du système formé des trois éléments optiques 502, 504, 506 qui vient d'être décrit :

$$\tan 2\alpha = \frac{\sin 2\alpha_2 - \sin 2\alpha_1}{\cos 2\alpha_2 - \cos 2\alpha_1} = -\frac{\cos(\alpha_1 + \alpha_2)}{\sin(\alpha_1 + \alpha_2)} \text{ (formule 1)}$$

$$C = C_0\left(\cos 2(\alpha - \alpha_2) - \cos 2(\alpha - \alpha_1)\right) \text{ (formule 2)}$$

$$S = S_r - \frac{C}{2} \text{ (formule 3).}$$

On remarque que le terme ($-C/2$) dans la formule 3 correspond à la puissance sphérique générée par la résultante des deux lentilles à puissance cylindrique 502, 504.

Grâce à sa structure présentant trois roues dentées, un tel ensemble optique peut facilement coopérer (lorsqu'il est monté sur des lunettes de compensation visuelle du type représenté en figure 4) avec un équipement de réception 400 tel que décrit plus haut.

En pilotant la position en rotation de la lentille plan-cylindre convexe 502 (au moyen du premier moteur d'entraînement 420) et la position en rotation de la lentille plan-cylindre concave 504 (au moyen du troisième moteur d'entraînement 440), indépendamment l'une de l'autre, comme décrit ci-après, on peut faire varier indépendamment chacun des angles $\alpha_1$, $\alpha_2$ de 0° à 360° et ainsi obtenir une puissance cylindrique C réglable entre -2.$C_0$ et 2.$C_0$ (soit ici entre -10D et 10D), et ce pour n'importe quel angle d'astigmatisme réglable entre 0° et 360° obtenu par une commande simultanée des deux lentilles. Comme l'indique la formule numéro 3, la résultante de puissance sphérique induite par la résultante de l'orientation des deux lentilles cylindriques 502, 504 est compensée à l'aide de la lentille sphérique de puissance variable 506.

On peut également prévoir dans ce contexte un mécanisme de synchronisation entre les deux lentilles cylindriques 502, 504 de façon à ce que celles-ci tournent en sens opposé d'un angle identique.

Par ailleurs, en faisant varier la puissance sphérique $S_v$ de la lentille sphérique 6 (à l'aide du second moteur d'entraînement 430), on peut régler la puissance sphérique S du système formé des trois lentilles 502, 504, 506.

## Revendications

1.  Equipement d'optométrie (400) comprenant :

    - un socle (410) conçu pour recevoir des lunettes de compensation visuelle (300), le socle (410) présentant un logement (402), dimensionné de manière à accueillir les lunettes de compensation visuelle (300), et des ouvertures (404), formées dans une paroi latérale du logement (402), pour le passage de branches (320) des lunettes de compensation visuelle (300) ;
    - au moins un élément de transmission (425 ; 435 ; 445) conçu pour coopérer avec un organe de réglage (352 ; 354 ; 356 ; 552 ; 554 ; 556) d'une caractéristique de correction optique desdites lunettes (300) ;
    - au moins un moteur d'entraînement (420 ; 430 ; 440) de l'élément de transmission (425 ; 435 ; 445).

2.  Equipement d'optométrie selon la revendication 1, dans lequel un module de communication (490) est conçu pour recevoir une consigne en provenance d'un appareil électronique extérieur et dans lequel un module de commande (480) est conçu pour commander le moteur d'entraînement (420 ; 430 ; 440) en fonction de la consigne reçue.

3.  Equipement d'optométrie selon la revendication 1 ou 2, comprenant un afficheur (450) et un imageur (455) disposés de part et d'autre d'une région (402) de réception des lunettes de compensation visuelle (300).

4.  Equipement d'optométrie selon la revendication 3, comprenant un module d'analyse (480) d'une image de l'afficheur générée par l'imageur (455) et un module de commande (480) du moteur d'entraînement (420 ; 430 ; 440) en fonction de données générées par le module d'analyse (480).

5.  Ensemble comprenant un équipement d'optométrie (400) et des lunettes de compensation visuelle (300), l'équipement d'optométrie (400) comprenant :

    - un socle (410) de réception desdites lunettes (300), le socle (410) présentant un logement (402), dimensionné de manière à accueillir les lunettes de compensation visuelle (300), et des ouvertures (404), formées dans une paroi latérale du logement (402), pour le passage de branches (320) des lunettes de compensation visuelle (300) ;
    - au moins un élément de transmission (425 ; 435 ; 445) conçu pour coopérer avec un organe de réglage (352 ; 354 ; 356 ; 552 ; 554 ; 556) d'une caractéristique de correction optique desdites lunettes (300) et
    - au moins un moteur d'entraînement (420 ; 430 ; 440) de l'élément de transmission (425 ; 435 ; 445).

6.  Ensemble selon la revendication 5, dans lequel les lunettes de compensation visuelle (300) comprennent au moins une lentille (350) ayant un axe optique et sont conçues pour modifier, en cas de mouvement de l'organe de réglage (356 ; 556), une puissance sphérique selon l'axe optique générée par ladite lentille (350).

7.  Ensemble selon la revendication 5, dans lequel les lunettes de compensation visuelle (300) comprennent au moins une lentille (350) ayant un axe optique et sont conçues pour modifier, en cas de mouvement de l'organe de réglage (352 ; 354 ; 552 ; 554), une correction cylindrique selon l'axe optique générée par ladite lentille (350).

8. Ensemble selon la revendication 7, dans lequel les lunettes de compensation visuelle (300) sont conçues pour modifier, en cas de mouvement de l'organe de réglage (354), une puissance de ladite correction cylindrique.

9. Ensemble selon la revendication 7, dans lequel les lunettes de compensation visuelle (300) sont conçues pour modifier, en cas de mouvement de l'organe de réglage (352), un axe de ladite correction cylindrique.

10. Système comprenant un équipement d'optométrie (400) conforme à la revendication 2 et ledit appareil électronique extérieur, **caractérisé en ce que** ledit appareil électronique extérieur est un autre appareil d'optométrie.

11. Système selon la revendication 10, dans lequel l'autre appareil d'optométrie est un auto-réfractomètre ou un réfracteur.

12. Système selon la revendication 10 ou 11, dans lequel ledit appareil électronique extérieur détermine ladite consigne en fonction d'une mesure d'amétropie ou d'astigmatisme.

**Patentansprüche**

1. Optometrievorrichtung (400) umfassend:

   - einen Sockel (410), der dafür ausgelegt ist, eine Sehausgleichsbrille (300) aufzunehmen, wobei der Sockel (410) eine Aufnahme (402), die so bemessen ist, dass sie die Sehausgleichsbrille (300) aufnimmt, und Öffnungen (404), die in einer Seitenwand der Aufnahme (402) ausgebildet sind, für den Durchtritt von Bügeln (320) der Sehausgleichsbrille (300) aufweist;
   - mindestens ein Übertragungselement (425; 435; 445), das dafür ausgelegt ist, mit einem Organ zur Einstellung (352; 354; 356; 552; 554; 556) einer optischen Korrektureigenschaft der Brille (300) zusammenzuwirken;
   - mindestens einen Antriebsmotor (420; 430; 440) des Übertragungselements (425; 435; 445).

2. Optometrievorrichtung nach Anspruch 1, wobei ein Kommunikationsmodul (490) dafür ausgelegt ist, eine Vorgabe von einem äußeren elektronischen Gerät zu empfangen, und wobei ein Steuerungsmodul (480) dafür ausgelegt ist, den Antriebsmotor (420; 430; 440) in Abhängigkeit von der empfangenen Vorgabe zu steuern.

3. Optometrievorrichtung nach Anspruch 1 oder 2, umfassend eine Anzeigeeinrichtung (450) und einen Bildgeber (455), die beiderseits eines Empfangsbereichs (402) der Sehausgleichsbrille (300) angeordnet sind.

4. Optometrievorrichtung nach Anspruch 3, umfassend ein Modul zur Analyse (480) eines Bilds der Anzeigeeinrichtung, das vom Bildgeber (455) erzeugt wird, und ein Steuerungsmodul (480) des Antriebsmotors (420; 430; 440) in Abhängigkeit von den Daten, die vom Analysemodul (480) erzeugt werden.

5. Anordnung umfassend eine Optometrievorrichtung (400) und eine Sehausgleichsbrille (300), wobei die Optometrievorrichtung (400) Folgendes umfasst:

   - einen Sockel (410) zur Aufnahme der Brille (300), wobei der Sockel (410) eine Aufnahme (402), die so bemessen ist, dass sie die Sehausgleichsbrille (300) aufnimmt, und Öffnungen (404), die in einer Seitenwand der Aufnahme (402) ausgebildet sind, für den Durchtritt von Bügeln (320) der Sehausgleichsbrille (300) aufweist;
   - mindestens ein Übertragungselement (425; 435; 445), das dafür ausgelegt ist, mit einem Organ zur Einstellung (352; 354; 356; 552; 554; 556) einer optischen Korrektureigenschaft der Brille (300) zusammenzuwirken, und
   - mindestens einen Antriebsmotor (420; 430; 440) des Übertragungselements (425; 435; 445).

6. Anordnung nach Anspruch 5, wobei die Sehausgleichsbrille (300) mindestens eine Linse (350) mit einer optischen Achse umfasst und dafür ausgelegt ist, bei Bewegung des Einstellungsorgans (356; 556) eine sphärische Stärke gemäß der optischen Achse, die von der Linse (350) erzeugt wird, zu ändern.

7. Anordnung nach Anspruch 5, wobei die Sehausgleichsbrille (300) mindestens eine Linse (350) mit einer optischen Achse umfasst und dafür ausgelegt ist, bei Bewegung des Einstellungsorgans (352; 354; 552; 554) eine Zylinderkorrektur gemäß der optischen Achse, die von der Linse (350) erzeugt wird, zu ändern.

8. Anordnung nach Anspruch 7, wobei die Sehausgleichsbrille (300) dafür ausgelegt ist, bei Bewegung des Einstellungsorgans (354) eine Stärke der Zylinderkorrektur zu ändern.

9. Anordnung nach Anspruch 7, wobei die Sehausgleichsbrille (300) dafür ausgelegt ist, bei Bewegung des Einstellungsorgans (352) eine Achse der Zylinderkorrektur zu ändern.

10. System umfassend eine Optometrievorrichtung (400) nach Anspruch 2 und das äußere elektronische Gerät, **dadurch gekennzeichnet, dass** das äußere elektronische Gerät ein anderes Optometriegerät ist.

11. System nach Anspruch 10, wobei das andere Optometriegerät ein Autorefraktometer oder ein Refraktor ist.

12. System nach Anspruch 10 oder 11, wobei das äußere elektronische Gerät die Vorgabe in Abhängigkeit von einer Ametropie- oder Astigmatismusmessung bestimmt.

**Claims**

1. Optometry apparatus (400) comprising:

   - a holder (410) designed to receive visual compensation spectacles (300), the holder (410) exhibiting a housing (402), dimensioned so as to accommodate the visual compensation spectacles (300), and openings (404), formed in a lateral wall of the housing (402), for the passage of temples (320) of the visual compensation spectacles (300);
   - at least one transmission element (425; 435; 445) designed to cooperate with a member (352; 354; 356; 552; 554; 556) for adjusting an optical correction characteristic of said spectacles (300);
   - at least one motor (420; 430; 440) for driving the transmission element (425; 435; 445).

2. Optometry apparatus according to Claim 1, in which a communication module (490) is designed to receive a setpoint originating from an exterior electronic device and in which a control module (480) is designed to control the driving motor (420; 430; 440) as a function of the setpoint received.

3. Optometry apparatus according to Claim 1 or 2, comprising a display (450) and an imager (455) which are disposed on either side of a region (402) of reception of the visual compensation spectacles (300).

4. Optometry apparatus according to Claim 3, comprising a module (480) for analysis of an image of the display, which image is generated by the imager (455), and a module (480) for control of the driving motor (420; 430; 440) as a function of data generated by the analysis module (480) .

5. Assembly comprising an optometry apparatus (400) and visual compensation spectacles (300), the optometry apparatus (400) comprising:

   - a holder (410) for receiving said spectacles (300), the holder (410) exhibiting a housing (402), dimensioned so as to accommodate the visual compensation spectacles (300), and openings (404), formed in a lateral wall of the housing (402), for the passage of temples (320) of the visual compensation spectacles (300);
   - at least one transmission element (425; 435; 445) designed to cooperate with a member (352; 354; 356; 552; 554; 556) for adjusting an optical correction characteristic of said spectacles (300) and
   - at least one motor (420; 430; 440) for driving the transmission element (425; 435; 445).

6. Assembly according to Claim 5, in which the visual compensation spectacles (300) comprise at least one lens (350) having an optical axis and are designed to modify, in case of movement of the adjusting member (356; 556), a spherical power along the optical axis, which power is generated by said lens (350).

7. Assembly according to Claim 5, in which the visual compensation spectacles (300) comprise at least one lens (350) having an optical axis and are designed to modify, in case of movement of the adjusting member (352; 354; 552; 554), a cylindrical correction along the optical axis, which correction is generated by said lens (350).

8. Assembly according to Claim 7, in which the visual compensation spectacles (300) are designed to modify, in case of movement of the adjusting member (354), a power of said cylindrical correction.

9. Assembly according to Claim 7, in which the visual compensation spectacles (300) are designed to modify, in case of movement of the adjusting member (352), an axis of said cylindrical correction.

10. System comprising an optometry apparatus (400) according to Claim 2 and said exterior electronic device, **characterized in that** said exterior electronic device is another optometry device.

11. System according to Claim 10, in which the other optometry device is an auto-refractometer or a refractor.

12. System according to Claim 10 or 11, in which said exterior electronic device determines said setpoint as a function of an ametropia or astigmatism measurement.

Fig.1

Fig.2

Fig.3

**Fig.4**

300
312
315
320
310
350
354
352
356
330
325

**Fig.5**

400
460
404
455
430
402
410
450
440
420
425
442
445
435

**Fig.6**

**Fig.7**

**Fig.8**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015107303 A
- EP 1138253 A
- US 8000022 B
- EP 2034338 A